# EUROPÄISCHE PATENTSCHRIFT

(11) **EP 2 412 324 B1**
(45) Veröffentlichungstag und Bekanntmachung des Hinweises auf die Patenterteilung: **30.05.2018**
(21) Anmeldenummer: 11005926.8
(22) Anmeldetag: 20.07.2011
(51) Int. Cl.: A61B 17/28, B25B 7/08

(54) **Medizinisches Handwerkzeug**
Medicinal hand-held tool
Outil manuel médical

(30) Priorität: 29.07.2010 DE 202010010843 U
(43) Veröffentlichungstag der Anmeldung: 01.02.2012
(73) Patentinhaber: Innovations Medical GmbH, 78532 Tuttlingen (DE)
(72) Erfinder: Kreidler, Winfried, 78532 Tuttlingen (DE)
(74) Vertreter: Binner, Bernhard

(56) Entgegenhaltungen:
- WO-A1-01/95812
- DE-C- 662 701
- DE-U1-202009 002 367
- GB-A- 2 280 397
- US-A- 168 012
- US-A- 1 510 416

## Beschreibung

Die Erfindung betrifft ein scherenartiges oder zangenartiges medizinisches Handwerkzeug mit einem ersten und einem zweiten Handhebel, welche jeweils einen Handgriff und ein Werkzeugteil aufweisen und welche über einen Gelenkzapfen schwenkbar miteinander in Verbindung stehen, wobei der erste Handhebel im Bereich zwischen seinem Handgriff und seinem Werkzeug eine abgeflachte erste Lagerplatte bildet und der zweite Handhebel im Bereich zwischen seinem Handgriff und seinem Werkzeug eine abgeflachte zweite Lagerplatte bildet, in deren Bereich der Gelenkzapfen angeordnet ist, wobei der Gelenkzapfen in der ersten Lagerplatte feststehend angeordnet ist und die zweite, mit einer Lagerbohrung versehene Lagerplatte schwenkbar aufnimmt. Solche Handwerkzeuge sind beispielsweise aus der US 168 012 A, DE 20 2009 002367 U1, GB 2 280 397 A, DE662 701 C und der WO 01/95812 A1 bekannt. Bei Handwerkzeugen der gattungsgemäßen Art liegen die beiden Lagerplatten der beiden Handhebel im normalen Betriebszustand flächig aufeinander. Dies hat zur Folge, dass manche Stellen, insbesondere im Bereich des die beiden Handhebel bzw. Lagerplatten miteinander verbindenden Gelenkzapfens, maschinell nicht vollständig und zuverlässig gereinigt werden können. Diese Bereiche um den Gelenkzapfen der flächig aufeinander liegenden Lagerplatten sind für Strahlen der Reinigungsflüssigkeit in einer Reinigungsmaschine nicht direkt zugänglich, sondern werden durch andere Teile verdeckt.

Der Erfindung liegt die Aufgabe zugrunde, ein Handwerkzeug der eingangs genannten Art so zu gestalten, dass es maschinell, z.B. in einer Flüssigkeitsstrahlen erzeugenden Reinigungsmaschine zuverlässig und vollständig gereinigt werden kann, d.h., dass keine "toten Räume" entstehen, in welche die Reinigungsstrahlen nicht direkt eindringen können.

Gelöst wird diese Aufgabe erfindungsgemäß durch die Merkmale des Anspruchs 1. Ein derart gestaltetes Handwerkzeug lässt sich in eine Öffnungsstellung bringen, in welcher die normalerweise kritischen Bereiche um den Gelenkzapfen freigelegt sind, so dass auch diese von den Reinigungsstrahlen erfasst und zuverlässig gereinigt werden können. Hierzu sind die beiden Handhebel in eine "geöffnete" Schwenkstellung bringbar, in welcher es möglich ist, die beiden Lagerplatten der Handhebel relativ zueinander in Richtung des Gelenkzapfens zu verstellen zw. zu verschieben, ohne dass das Handwerkzeug auseinander fallen kann. Hierzu ist der Gelenkzapfen einerseits feststehend mit der ersten Lagerplatte des ersten Handhebels verbunden. Die zweite Lagerplatte des zweiten Handhebels ist über ihre Lagerbohrung auf dem Gelenkzapfen relativ zur ersten Lagerplatte bzw. Handhebel schwenkbar aufgenommen. Um sicherzustellen, dass die zweite Lagerplatte in der geöffneten Schwenkstellung nicht vom Gelenkzapfen abgezogen werden kann oder versehentlich von diesem abrutscht, weist der Gelenkzapfen an seinem freien Ende einen radial vorstehenden Ringflansch auf, der beim Verschieben der zweiten Lagerplatte entlang des Gelenkzapfens als eine Art Anschlag dient. Um sicherzustellen, dass die beiden Lagerplatten für die Zugänglichkeit mit Reinigungsflüssigkeit einen ausreichenden Abstand voneinander aufweisen, ist der Abstand zwischen der ersten Lagerplatte, welche den feststehenden Gelenkzapfen aufweist und dem Ringflansch zumindest doppelt so groß wie die Dicke der zweiten Lagerplatte gewählt.

Um einen möglichst ausreichenden Verschiebeweg sicherzustellen, jedoch einen störenden "Überstand" des Gelenkzapfens bei in ihrer Betriebsstellung aufeinander liegenden Lagerplatte zu vermeiden, kann die Ausgestaltung nach Anspruch 2 vorgesehen sein. Danach ist vorgesehen, dass die Lagerbohrung der zweiten Lagerplatte des zweiten, auf dem Gelenkzapfen gelagerten Handhebels außenseitig mit einer radial erweiterten, den Ringflansch des Gelenkzapfens aufnehmenden Bohrungserweiterung versehen ist. Wird die zweite Lagerplatte des zweiten Handhebels entlang des Gelenkzapfens geschoben, so gelangt der Ringflansch in den Bereich dieser Bohrungserweiterung, in welcher der Ringflansch bei maximalem "Verschiebeweg" teilweise oder vollständig aufgenommen wird. Mit der Ausgestaltung nach Anspruch 2 besteht die Möglichkeit, den Gelenkzapfen kurz zu halten, damit der Gelenkzapfen bei Gebrauch des Handwerkzeugs nicht zu weit aus der zweiten Lagerplatte des zweiten Handhebels herausragt. Erfindungsgemäß ergibt sich der handhabungstechnische Vorteil, die beiden Handhebel in ihrer maximalen Schwenkstellung gegeneinander verriegeln zu können, damit sie diese Schwenkstellung auch während des Reinigungsvorgangs in der Reinigungsmaschine beibehalten. Danach ist vorgesehen, dass der erste Handhebel im Bereich seines Werkzeuges eine außenseitig in Richtung des Gelenkzapfens vorstehende, im Schwenkbereich der zweiten Lagerplatte des zweiten Handhebels liegende Sperrfläche aufweist und, dass die zweite Lagerplatte mit einem Kantenbereich in ihrer von der ersten Lagerplatte abgehobenen Position an der Sperrfläche abstützbar ist.

Die Ausgestaltungen nach den Ansprüchen 4 und 5 erleichtern das gegenseitige Verriegeln der beiden Handhebel in ihrer maximalen Schwenkstellung.

So ist gemäß Anspruch 3 vorgesehen, dass die Sperrfläche im Bereich einer Ebene liegt, in welcher sich die der ersten Lagerplatte zugewandte, innere Kontaktfläche der zweiten Lagerplatte des zweiten Handhebels befindet, wenn sich die zweite Lagerplatte auf dem Gelenkzapfen in ihrer am weitesten von der ersten Lagerplatte des ersten Handhebels entfernten Lage befindet.

Weiter ist hierzu gemäß Anspruch 4 vorgesehen, dass die Sperrfläche mit einer schrägen Führungskante versehen ist.

Gemäß Anspruch 5 kann vorgesehen sein, dass die Lagerplatten eine zumindest annähernd deckungsgleiche Flächenform aufweisen und im Wesentlichen gleich dick sind. Durch diese deckungsgleiche Formgebung bilden die Lagerplatten, insbesondere im geschlossenen Zustand der Handhebel, eine einheitliche Außenkontur.

Weiter kann gemäß Anspruch 6 im Umgebungsbereich der Bohrungserweiterung ein in Richtung des Gelenkzapfens vorstehender, umlaufender Ringsteg vorgesehen sein, in welchem sich die Bohrungserweiterung fortsetzt. Dieser Ringsteg endet bei aufeinander liegenden Lagerplatten im Bereich des Ringflansches des Gelenkzapfens. Durch diese Ausgestaltung wird der Gelenkzapfen mit seinem Ringflansch auch in der geschlossenen Schwenklage der beiden Handhebel, bei aufeinander liegenden Lagerplatten in der Bohrungserweiterung zumindest teilweise aufgenommen. Damit wird die Verletzungsgefahr beispielsweise durch "Einklemmen" eines Fingers einer Bedienperson beim Verschieben der zweiten Lagerplatte entlang des Gelenkzapfens erheblich vermindert.

Im Folgenden wird die Erfindung anhand der beiliegenden Zeichnungen näher erläutert. Es zeigt:
- Fig. 1: eine perspektivische Darstellung eines zangenartigen medizinischen Handwerkzeuges in geschlossenem Zustand;
- Fig. 2: eine vergrößerte perspektivische Teilansicht des Handwerkzeugs aus Fig. 1 mit sich in ihrer geöffneten Schwenkstellung befindenden Handhebeln bei "noch" aufeinander liegenden Lagerplatten;
- Fig. 3: die Teilansicht aus Fig. 2 mit entlang des Gelenkzapfens "verschobenen" Lagerplatte der Handhebel;
- Fig. 4: einen Teilschnitt des Handwerkzeuges aus Fig. 1 durch den Gelenkzapfen bei geschlossenem Handwerkzeug;
- Fig. 5: den Teilschnitt aus Fig. 4 bei sich in ihrer geöffneten Schwenkstellung befindenden Handhebel und auseinander "geschobenen" Lagerplatten ;
- Fig. 6: eine teilweise Draufsicht auf das Handwerkzeug aus Fig. 1, welches in seiner geöffneten Schwenkstellung mit einer Haltevorrichtung in Eingriff steht, wobei die Haltevorrichtung im Schnitt VI - VI aus Fig. 7 dargestellt ist;
- Fig. 7: eine Seitenansicht VII aus Fig. 6 des Handwerkzeuges zusammen mit der Aufhängeeinrichtung;
- Fig. 8: eine perspektivische Teilansicht eines Handwerkzeuges mit einem außenseitig auf der zweiten Lagerplatte im Umgebungsbereich des Gelenkzapfens vorstehenden Ringsteg;
- Fig. 9: einen Teilschnitt des Handwerkzeuges aus Fig. 8 durch den Gelenkzapfen bei geschlossenem Handwerkzeug.

Wie aus den Zeichnungen ersichtlich ist, handelt es sich bei dem dargestellten Ausführungsbeispiel eines Handwerkzeuges 1 um eine Art Flachzange. Die Erfindung ist allerdings nicht auf diese Art von "Handwerkzeug" beschränkt, sondern erstreckt sich auch auf andere Handwerkzeuge, welche zwei relativ zueinander schwenkbare Handhebel aufweisen. So könnte das Handwerkzeug 1 beispielsweise auch als Schere mit zwei Scherklingen oder als Kneifzange mit zwei quer verlaufenden Schneidkanten ausgebildet sein. Auch andere Ausbildungen für andere Anwendungszwecke sind denkbar.

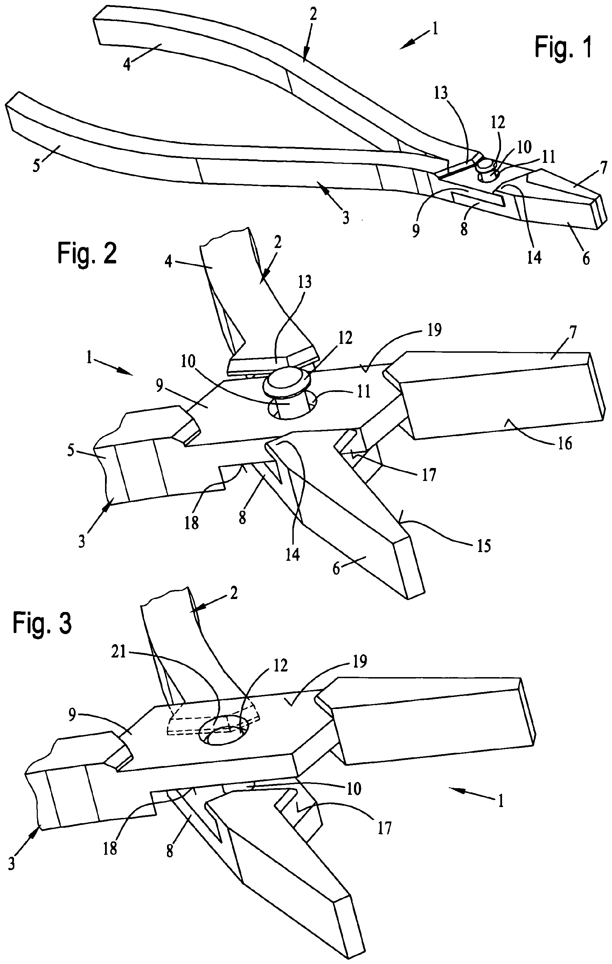

Das in Fig. 1 isometrisch dargestellte Handwerkzeug 1 besteht aus einem ersten Handhebel 2 und einem zweiten Handhebel 3. Der erste Handhebel 2 ist mit einem ersten Handgriff 4 und der zweite Handhebel 3 mit einem zweiten Handgriff 5 versehen. Diesen Handgriffen 4 und 5 ist jeweils in Längserstreckung der Handhebel 2 bzw. 3 ein erstes bzw. zweites Werkzeug jeweils in Form einer ersten bzw. zweiten Klemmbacke 6 bzw. 7 zugeordnet. Der erste Handhebel 2 weist zwischen seinem Handgriff 4 und seiner Klemmbacke 6 eine abgeflachte erste Lagerplatte 8 auf, während zwischen dem zweiten Handgriff 5 und der Klemmbacke 7 des zweiten Handhebels 3 eine zweite abgeflachte Lagerplatte 9 vorgesehen ist. Dies beiden Lagerplatten 8 und 9 können unterschiedlich dick ausgebildet sein. Bei der vorliegenden Ausführungsvariante weisen beiden Lagerplatten 8, 9 jedoch etwa dieselbe Dicke auf. In dem in Fig. 1 dargestellten, montierten Zustand der beiden Handhebel 2 und 3 und in der dargestellten "geschlossenen" Schwenklage der beiden Handhebel 2 und 3 liegen die erste Lagerplatte 8 und die zweite Lagerplatte 9 flächig aufeinander.

Im Bereich dieser beiden Lagerplatten 8 und 9 ist ein Gelenkzapfen 10 vorgesehen, über welchen die Lagerplatten 8, 9 und somit die beiden Handhebel 2 und 3 relativ zueinander und schwenkbar miteinander verbunden sind. Der Gelenkzapfen 10 ist bei der dargestellten Ausführungsvariante feststehend mit der ersten Lagerplatte 8 verbunden und durchragt eine entsprechende Lagerbohrung 11 der zweiten Lagerplatte 9, wobei diese Lagerbohrung 11 in Fig. 1 nur andeutungsweise erkennbar ist. Weiter ist aus Fig. 1 ersichtlich, dass der Gelenkzapfen 10 die zweite Lagerplatte 9 nach außen hin überragt und in seinem freien Endbereich einen radial erweiterten Ringflansch 12 aufweist.

In der in Fig. 1 dargestellten geschlossenen Schwenklage der beiden Handhebel 2 und 3 hintergreift die zweite Lagerplatte 9 zwei sich relativ zum Gelenkzapfen 10 gegenüber liegende Sicherungsstege 13 und 14, welche entsprechend der Dicke der zweiten Lagerplatte 9 einen Abstand zur ersten Lagerplatte 8 aufweisen und parallel zu dieser verlaufen. Der erste Sicherungssteg 13 ist dabei im Übergangsbereich von der ersten Lagerplatte 8 zum ersten Handgriff 4 und der zweite Sicherungssteg 14 im Übergangsbereich von der ersten Lagerplatte 8 zur ersten Klemmbacke 6 angeordnet. Es ist erkennbar, dass die zweite Lagerplatte 9 in dieser geschlossenen Schwenkstellung mit geringem Spiel zwischen den beiden Sicherungsstegen 13, 14 und der ersten Lagerplatte 8 aufgenommen ist. Durch diese Ausgestaltung ist sichergestellt, dass die zweite Lagerplatte 9 im "normalen" Betrieb des Handwerkzeuges 1 nicht von der ersten Lagerplatte 8 in Richtung des Gelenkzapfens 10 abgehoben werden kann, so dass die Handgriffe 4 und 5 sowie die beiden Klemmbacken 6 und 7 in einer im Wesentlichen gemeinsamen Ebene liegend gegeneinander schwenkbar sind. Um das Handwerkzeug 1 möglichst optimal reinigen zu können, ist es notwendig, insbesondere die beiden einander zugewandten Kontaktflächen der aufeinander "liegenden" Lagerplatten 8 und 9 zugänglich zu machen. Hierzu ist das Handwerkzeug 1 mit seinen Handhebeln 2 und 3 aus der in Fig. 1 dargestellten geschlossenen Schwenklage in eine geöffnete Schwenklage zu bringen.

Fig. 2 zeigt hierzu eine vergrößerte, perspektivische Darstellung des Handwerkzeuges 1, wobei die beiden Handgriffe 4 und 5 des ersten und zweiten Handhebels 2 und 3 jeweils verkürzt dargestellt sind. Der erste und zweite Handhebel 2 und 3 befinden sich in Fig. 2 in einer "geöffneten" Schwenklage, in welcher die erste und die zweite Klemmbacke 6 und 7 mit ihren einander zugewandten Klemmflächen 15 und 16 einen Abstand voneinander aufweisen. Die Anordnung des Gelenkzapfens 10 in den beiden Lagerplatten 8 und 9 ist so gewählt, dass die flachen Klemmflächen 15 und 16 der beiden Klemmbacken 6 und 7 bei entsprechender Betätigung der Hangriffe 4 und 5 gegeneinander gepresst werden können.

In der in Fig. 2 dargestellten "geöffneten" Schwenklage der beiden Handhebel 2 und 3 sind die beiden Klemmflächen 15 und 16 der Klammbacken 6 und 7 bereits frei zugänglich, so dass diese mit einer entsprechenden Reinigungsflüssigkeit beaufschlagt werden können. Die zweite Lagerplatte 9 ist relativ zur ersten Lagerplatte 8 verschwenkt, so dass sich die erste "Kontaktfläche" 17 der ersten Lagerplatte 8 und die zweite "Kontaktfläche" 18 der zweiten Lagerplatte 9 gegenseitig teilweise abdecken. Folglich können die "noch" abgedeckten Bereiche der Kontaktflächen 17 und 18 nicht oder zumindest nicht ausreichend mit Reinigungsflüssigkeit beaufschlagt werden.

Um nun auch diese Bereiche der Kontaktflächen 17 und 18 der ersten Lagerplatte 8 und der zweiten Lagerplatte 9 zugänglich machen zu können, ist der Gelenkzapfen 10 mit seinem radial erweiterten Ringflansch 12 vorgesehen. Wie bereits zu Fig. 1 erwähnt, ragt der Gelenkzapfen 10 durch die Lagerbohrung 11 der zweiten Lagerplatte 9 hindurch, so dass der Ringflansch zur Kontaktfläche 17 der ersten Lagerplatte 8 und insbesondere auch zur "äußern" Oberfläche 19 der zweiten Lagerplatte 9 einen gewissen Abstand aufweist.

Weiter ist aus Fig. 2 ersichtlich, dass in der geöffneten Schwenklage die zweite Lagerplatte 9 mit den beiden Sicherungsstegen 13 und 14 außer Eingriff steht. Damit ist es nun möglich, aufgrund des Abstandes des radial erweiterten Ringflansches 12 von der Oberfläche 19 der zweiten Lagerplatte 9 den zweiten Handhebel 3 in Richtung des Pfeiles 20 bzw. in Richtung des Gelenkzapfens 10 von der Kontaktfläche 17 der ersten Lagerplatte 8 abzuheben.

Einen solchen abgehobenen Zustand zeigt Fig. 3. Dabei ist aus Fig. 3 erkennbar, dass die zweite Lagerplatte 9 von der ersten Lagerplatte 8 abgehoben ist, wobei bei der dargestellten Ausführungsvariante der Gelenkzapfen 10 mit seinem radial erweiterten Ringflansch 12 in einer radial erweiterten Bohrungserweiterung 21 der in Fig. 3 nicht weiter erkennbaren Lagerbohrung aufgenommen ist. Die Bohrungserweiterung 21 ist als eine Art Einsenkung in der Oberfläche 19 der zweiten Lagerplatte 9 eingelassen. Somit ist aus Fig. 3 erkennbar, dass der Gelenkzapfen 10 mit seinem unteren, zur ersten Lagerplatte 8 hin aus der zweiten Lagerplatte 9 vorstehenden Endbereich sowie die beiden Kontaktflächen 17 und 18 der Lagerplatten 8 und 9 frei zugänglich sind.

Durch die radial erweiterte Ausgestaltung des Ringflansches 12 des Gelenkzapfens 10 wird ein weiteres "Auseinanderschieben" der beiden Lagerplatten 8 und 9 verhindert, so dass das Handwerkzeug 1 in der in Fig. 3 dargestellten Position gereinigt werden kann, ohne dass das Handwerkzeug 1 mit seinen Handhebeln 2 und 3 versehentlich voneinander trennbar sind.

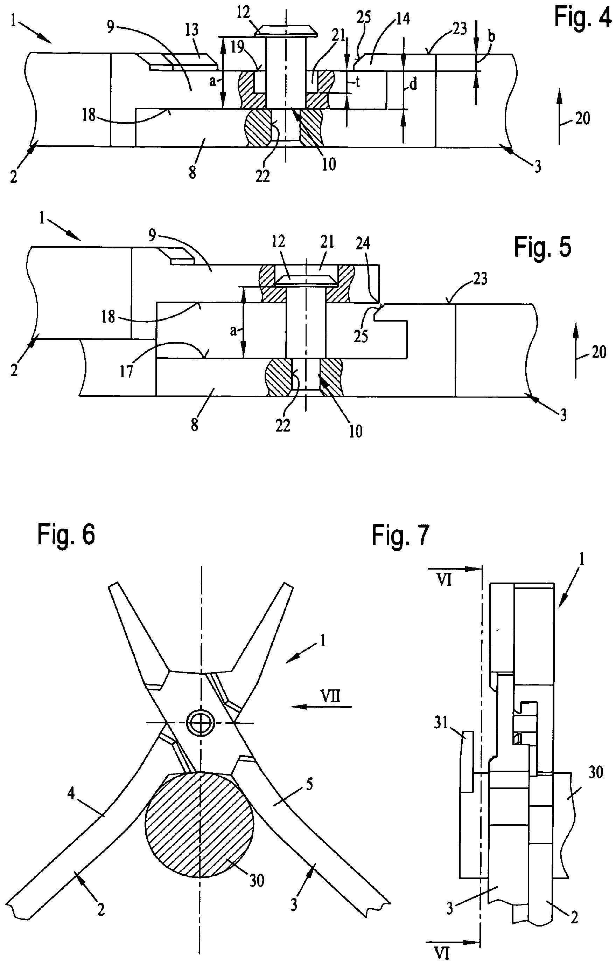

Hierzu zeigt Fig. 4 eine teilweise Seitenansicht des Handwerkzeuges 1 im Bereich der beiden Lagerplatten 8 und 9, welche in Fig. 4 im Bereich des Gelenkzapfens 10 teilweise geschnitten dargestellt sind. Die beiden andeutungsweise erkennbaren Handhebel 2 und 3 befinden sich in ihrer geschlossenen Schwenklage, wie diese in perspektivischer Darstellung aus Fig. 1 erkennbar ist.

Aus Fig. 4 ist ersichtlich, dass die zweite Lagerplatte 9 die beiden Sicherungsstege 13 und 14 hintergreift, so dass ein Abheben in Richtung des Pfeiles 20 in dieser in Fig. 4 dargestellten Betriebsstellung sicher verhindert wird.

Der Gelenkzapfen 10 ist in eine Aufnahmebohrung 22 der ersten Lagerplatte 8 festsitzend eingesetzt und durchragt die zweite Lagerplatte 9 vertikal nach oben. Dabei kann der Gelenkzapfen 10 in der Aufnahmebohrung 22 beispielsweise verschweißt, verlötet, verklebt oder auch feststehend eingeschraubt sein. Die "axiale" Länge des Gelenkzapfens 10 ist derart gewählt, dass der Abstand **a** zwischen der ersten Lagerplatte 8 und dem Ringsflansch 12 etwa der doppelten der Dicke **d** der zweiten Lagerplatte 9 entspricht. Der Ringflansch 12 weist bei der dargestellten Ausführungsvariante der Fig. 4 zur Oberfläche 19 der zweiten Lagerplatte 9 in der dargestellten, geschlossenen Schwenklage der beiden Handhebel 2 und 3 einen Abstand auf, welcher kleiner ist als die Dicke **d** der zweiten Lagerplatte 9.

Die ebenfalls in Fig. 4 ersichtliche, als Einsenkung ausgebildete Bohrungserweiterung 21, ist in ihrer "Tiefe" t etwas größer ausgebildet als die Dicke **b** der beiden Sicherungsstege 13 und 14. Dadurch wird erreicht, dass bei einer Verschiebung der zweiten Lagerplatte 9 in Richtung des Pfeiles 20 entlang des Gelenkzapfens 10, die untere Kontaktfläche 18 der zweiten Lagerplatte 9 im vollständig auseinander geschobenen Zustand der beiden Lagerplatten 8 und 9 etwa im Bereich der Oberfläche 23 des Sicherungssteges 14 liegt, wie dies aus Fig. 5 erkennbar ist. Durch eine relative Schwenkbewegungen der zweiten Lagerplatte 9 zur ersten Lagerplatte 8 kann die Kontaktfläche 18 mit dieser Oberfläche 23 in Eingriff gebracht werden, so dass diese als Sperrfläche wirkt und somit verhindert wird, dass die beiden Lagerplatten 8 und 9 unbeabsichtigt wieder zusammen geschoben werden. Die Sperrfläche 23 kann zur "Arretierung" der beiden Lagerplatten 8, 9 einen erhöhten Reibungskoeffizienten aufweisen oder mit Sperrzähen und/oder auch mit Rastmitteln versehen sein. Um die beiden Handhebel 2, 3 in diese Verriegelungsposition bringen zu können, kann an der "Vorderkante" des Sicherungssteges 14 eine schräg geneigt verlaufende Führungskante 25 vorgesehen sein. Auf Grund dieser "Verriegelung" ist es auch möglich, das Handwerkzeug 1 in jeder beliebigen anderen Position im Strahlenbereich einer Reinigungsmaschine zu lagern, beispielsweise frei aufzuhängen.

In Fig. 5 ist diese auseinander geschobene Position der beiden Lagerplatten 8 und 9 erkennbar, wobei in Fig. 5 die entsprechenden Bezugzeichen eingetragen sind. In Fig. 5 befinden sich die beiden Handhebel 2 und 3 "noch nicht" in ihrer Verriegelungsposition. In dieser in Fig. 5 dargestellten abgehobenen Stellung der beiden Lagerplatten 8 und 9 sind deren Kontaktflächen 17 und 18 sowie der sich zwischen den beiden Lagerplatte 8 und 9 befindende Teil des Gelenkzapfens 10 frei zugänglich. Somit können diese Teile in einer Flüssigkeitsstrahlen erzeugenden Reinigungsmaschine maschinell gründlich gereinigt werden. Durch den radial vorstehenden Ringsflansch 12 des Gelenkzapfens 10 wird dabei ein "Auseinanderfallen" der beiden Handhebel 2 und 3 durch die Strahleinwirkung sicher verhindert.

Um die beiden Handhebel 2 und 3 in ihrer "Reinigungsposition" zu halten, kann das Handwerkzeug 1 beispielsweise mit seinen beiden Handgriffen 4 und 5 auch auf eine Haltevorrichtung 30 aufgesetzt werden, so dass ein versehentliches Schließen der beiden Handhebel 2 und 3 während des Reinigungsvorganges ebenfalls sicher ausgeschlossen ist. Hierzu zeigen die Fig. 6 und 7 beispielhaft einen solchen Eingriff des Handwerkzeuges 1 mit einer solchen Haltevorrichtung 30. Aus Fig. 7 ist hierbei erkennbar, dass diese Haltevorrichtung 30 in ihrem freien Endbereich einen vertikal nach oben stehenden Sicherungssteg 31 aufweisen kann. Diese Haltevorrichtung 30 kann in einem nicht weiter dargestellten Reinigungsraum einer Reinigungsmaschine angeordnet sein.

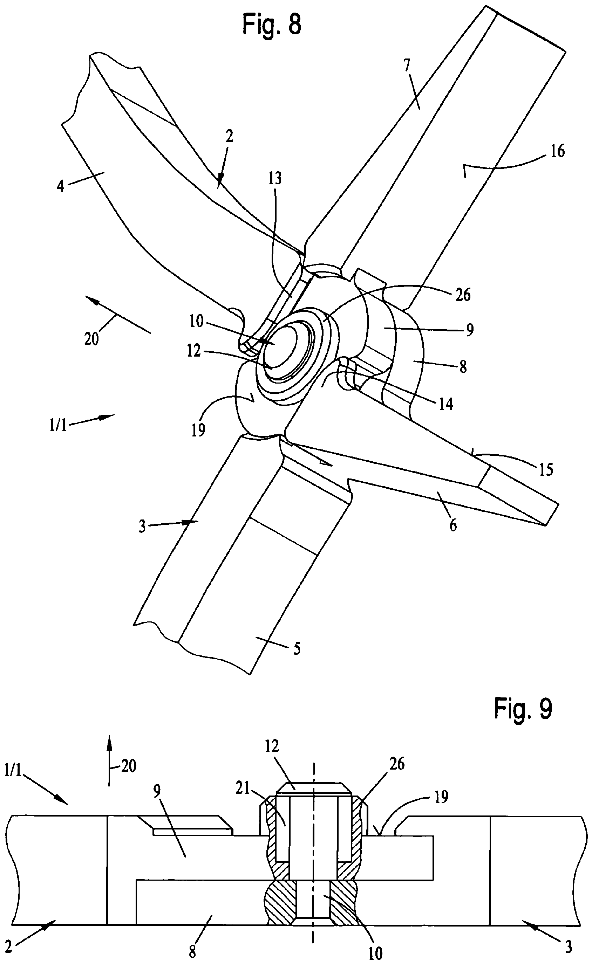

Die Fig. 8 und 9 zeigen Darstellungen einer zweiten Ausführungsvariante eines Handwerkzeuges 1/1, welches ebenfalls aus zwei gegeneinander schwenkbaren Handhebeln 2 und 3 gebildet ist. Die beiden Handgriffe 4 und 5 der beiden Handhebel 2 und 3 sind in Fig. 8 verkürzt dargestellt. Ebenfalls weisen die beiden Handhebel 2 und 3 jeweils ein Werkzeug in Form einer Klemmbacke 6 bzw. 7 auf, welche mit entsprechenden Klemmflächen 15 und 16 versehen sind. Das Handwerkzeug 1/1 aus Fig. 8 befindet sich in derselben Schwenklage wie dies für das Handwerkzeug 1 zu Fig. 2 für die dortige Ausführungsvariante 1 dargestellt ist. Weiter weist auch die Ausführungsvariante des Handwerkzeuges 1/1 aus Fig. 8 eine erste Lagerplatte 8 und eine zweite Lagerplatte 9 auf. Im geschlossenen Zustand der Handhebel 2 und 3 hintergreift die zweite Lagerplatte 9 ebenfalls zwei Sicherungsstege 13 und 14, wie dies für die Ausführungsvariante des Handwerkzeuges 1/1 der Fig. 1 dargestellt ist.

Die Ausführungsvariante des Handwerkzeuges 1/1 der Fig. 8 unterscheidet sich von der Ausführungsvariante 1 der Fig. 1 dadurch, dass die beiden Lagerplatten 8 und 9 jeweils eine Art kreisbogenförmig ausgebildete Grundstruktur aufweisen. Ansonsten ist die Funktionsweise identisch mit der Ausführungsvariante 1 der Fig. 1 bis 5, so dass in der in Fig. 8 dargestellten Schwenkposition der beiden Handhebel 2 und 3 zueinander die zweite Lagerplatte 9 ebenfalls von der ersten Lagerplatte 8 in Richtung des Pfeiles 20 abgehoben werden kann.

Der Umgebungsbereich des Ringflansches 12, des in Fig. 8 nur andeutungsweise erkennbaren Gelenkzapfens 10, weist einen umlaufenden, über die äußere Oberfläche 19 der zweiten Lagerplatte 9 vorstehenden Ringsteg 26 auf.

Dieser Ringsteg 26 ist in seiner Höhe derart ausgebildet, dass der Ringflansch 12 des Gelenkzapfens 10 (siehe Fig. 9) von der sich bis in den Ringsteg 26 fortsetzenden Bohrungserweiterung 21 zumindest teilweise aufgenommen wird. Durch diesen Ringsteg 26 wird insbesondere beim Verschieben der zweiten Lagerplatte 9 relativ zur ersten Lagerplatte 8 in Richtung des Pfeiles 20 die Verletzungsgefahr, beispielsweise durch Einklemmen eines Fingers, erheblich vermindert.

## Patentansprüche

1. Scherenartiges oder zangenartiges medizinisches Handwerkzeug (1, 1/1) mit einem ersten und einem zweiten Handhebel (2, 3), welche jeweils einen Handgriff (4, 5) und ein Werkzeugteil (6, 7) aufweisen und welche über einen Gelenkzapfen (10) schwenkbar miteinander in Verbindung stehen, wobei der erste Handhebel (2) im Bereich zwischen seinem Handgriff (4) und seinem Werkzeug (6) eine abgeflachte erste Lagerplatte (8) bildet und der zweite Handhebel (3) im Bereich zwischen seinem Handgriff (5) und seinem Werkzeug (7) eine abgeflachte zweite Lagerplatte (9) bildet, in deren Bereich der Gelenkzapfen (10) angeordnet ist, wobei der Gelenkzapfen (10) in der ersten Lagerplatte (8) feststehend angeordnet ist und die zweite mit einer Lagerbohrung (11) versehene Lagerplatte (9) schwenkbar aufnimmt, wobei der Gelenkzapfen (10) die zweite Lagerplatte (9) durchragt und in seinem die zweite Lagerplatte (9) durchragenden Endbereich mit einem radial erweiterten Ringflansch (12) versehen ist, der von der ersten Lagerplatte (8) einen Abstand (a) aufweist, welcher der doppelten Dicke (d) der auf dem Gelenkzapfen (10) gelagerten zweiten Lagerplatte (9) entspricht oder größer ist als die doppelte Dicke (d) der zweiten Lagerplatte (9), so dass der zweite Handhebel (3) in einer geöffneten Schwenklage zum ersten Handhebel (2) entlang des Gelenkzapfens (10) in eine Lage verschiebbar ist, in welcher die einander gegenüber liegenden Kontaktflächen (17, 18) der abgeflachten Lagerplatten (8, 9) frei liegen und
dass der erste Handhebel (2) im Bereich seines Werkzeuges (6) eine außenseitig in Richtung des Gelenkzapfens (10) vorstehende, im Schwenkbereich der zweiten Lagerplatte (9) des zweiten Handhebels (3) liegende Sperrfläche (23) aufweist und,
dass die zweite Lagerplatte (9) mit einem Kantenbereich (24) in ihrer von der ersten Lagerplatte (8) abgehobenen Position an der Sperrfläche (23) abstützbar ist.

2. Handwerkzeug nach Anspruch 1, **dadurch gekennzeichnet, dass** die Lagerbohrung (11) der zweiten Lagerplatte (9) des zweiten, auf dem Gelenkzapfen (10) gelagerten Handhebels (3) außenseitig mit einer radial erweiterten, den Ringflansch (12) des Gelenkzapfens (10)aufnehmenden Bohrungserweiterung (21) versehen ist.

3. Handwerkzeug nach Anspruch 1, **dadurch gekennzeichnet, dass** die Sperrfläche (23) im Bereich einer Ebene liegt, in welcher sich die der ersten Lagerplatte (8) zugewandte, innere Kontaktfläche (18) der zweiten Lagerplatte (9) des zweiten Handhebels (3) befindet, wenn sich die zweite Lagerplatte (9) auf dem Gelenkzapfen (10) in ihrer am weitesten von der ersten Lagerplatte (8) des ersten Handhebels (2) entfernten Lage befindet.

4. Handwerkzeug nach Anspruch 1 oder 3, **dadurch gekennzeichnet, dass** die Sperrfläche (23) mit einer schrägen Führungskante (25) versehen ist.

5. Handwerkzeug nach einem der Ansprüche 1 bis 4, **dadurch gekennzeichnet, dass** die Lagerplatten (8, 9) eine zumindest annähernd deckungsgleiche Flächenform aufweisen und im Wesentlichen gleich dick sind.

6. Handwerkzeug nach einem der Ansprüche 2 bis 5, **dadurch gekennzeichnet, dass** im Umgebungsbereich der Bohrungserweiterung (21) ein in Richtung des Gelenkzapfens (10) vorstehender, umlaufender Ringsteg (26) vorgesehen ist, in welchem sich die Bohrungserweiterung (21) fortsetzt und,
dass der Ringsteg (26) bei aufeinander liegenden Lagerplatten (8, 9) im Bereich des Ringflansches (12) des Gelenkzapfens (10) endet.

## Claims

1. Scissor-like or forceps-like medical hand tool (1, 1/1) with a first and a second hand lever (2, 3) which each have a handle (4, 5) and a tool part (6, 7) and which are connected to each other pivotably via a hinge pin (10), wherein the first hand lever (2), in the region between its handle (4) and its tool (6), forms a flat first bearing plate (8), and the second hand lever (3), in the region between its handle (5) and its tool (7), forms a flat second bearing plate (9) in the region of which the hinge pin (10) is arranged, wherein the hinge pin (10) is arranged fixedly in the first bearing plate (8) and pivotably receives the second bearing plate (9) provided with a bearing bore (11), wherein the hinge pin (10) passes through the second bearing plate (9) and, in its end region passing through the second bearing plate (9), is provided with a radially widened annular flange (12) which is at a distance (a), from the first bearing plate (8), that corresponds to twice the thickness (d) of the second bearing plate (9) mounted on the hinge pin (10) or that is greater than twice the thickness (d) of the second bearing plate (9), such that the second hand lever (3), in an opened pivoting position relative to the first hand lever (2), is movable along the hinge pin (10) to a position in which the mutually opposite contact surfaces (17, 18) of the flat bearing plates (8, 9) lie free, and in that the first hand lever (2), in the region of its tool (6), has a blocking surface (23) which protrudes externally in the direction of the hinge pin (10) and lies in the pivot range of the second bearing plate (9) of the second hand lever (3), and in that the second bearing plate (9), in its position raised from the first bearing plate (8), can be supported with an edge region (24) on the blocking surface (23).

2. Hand tool according to Claim 1, **characterized in that** the bearing bore (11) of the second bearing plate (9) of the second hand lever (3) mounted on the hinge pin (10) is provided externally with a radially widened bore widening (21) that receives the annular flange (12) of the hinge pin (10).

3. Hand tool according to Claim 1, **characterized in that** the blocking surface (23) lies in the region of a plane in which the inner contact surface (18) of the second bearing plate (9) of the second hand lever (3), facing towards the first bearing plate (8), is located when the second bearing plate (9) is located on the hinge pin (10) in its position farthest removed from the first bearing plate (8) of the first hand lever (2).

4. Hand tool according to Claim 1 or 3, **characterized in that** the blocking surface (23) is provided with an oblique guiding edge (25).

5. Hand tool according to one of Claims 1 to 4, **characterized in that** the bearing plates (8, 9) have an at least approximately congruent surface shape and are of substantially the same thickness.

6. Hand tool according to one of Claims 2 to 5, **characterized in that** a peripheral annular web (26) protruding in the direction of the hinge pin (10) is provided in the region surrounding the bore widening (21), in which annular web (26) the bore widening (21) continues, and **in that** the annular web (26) ends in the region of the annular flange (12) of the hinge pin (10) when the bearing plates (8, 9) lie on each other.

## Revendications

1. Outil manuel médical de type ciseaux ou pince (1, 1/1), comprenant un premier et un deuxième levier manuel (2, 3) qui présentent chacun une poignée (4, 5) et une partie d'outil (6, 7) et qui sont en liaison l'un avec l'autre de manière pivotante par le biais d'un goujon d'articulation (10), le premier levier manuel (2) formant, dans la région entre sa poignée (4) et son outil (6), une première plaque de support aplatie (8) et le deuxième levier manuel (3) formant, dans la région entre sa poignée (5) et son outil (7), une deuxième plaque de support aplatie (9), dans la région desquelles est disposé le goujon d'articulation (10), le goujon d'articulation (10) étant disposé de manière fixée dans la première plaque de support (8) et recevant de manière pivotante la deuxième plaque de support (9) pourvue d'un alésage de palier (11),
le goujon d'articulation (10) traversant la deuxième plaque de support (9) et étant pourvu, dans sa région d'extrémité traversant la deuxième plaque de support (9), d'une bride annulaire élargie radialement (12) qui est éloignée de la première plaque de support (8) d'une distance (a) qui correspond au double de l'épaisseur (d) de la deuxième plaque de support (9) supportée sur le goujon d'articulation (10) ou qui est supérieure au double de l'épaisseur (d) de la deuxième plaque de support (9), de telle sorte que le deuxième levier manuel (3), dans une position de pivotement ouverte par rapport au premier levier manuel (2), puisse être déplacé le long du goujon d'articulation (10) dans une position dans laquelle les surfaces de contact mutuellement opposées (17, 18) des plaques de support aplaties (8, 9) sont exposées et que le premier levier manuel (2), dans la région de son outil (6), présente une surface de blocage (23) faisant saillie du côté extérieur dans la direction du goujon d'articulation (10), située dans la région de pivotement de la deuxième plaque de support (9) du deuxième levier manuel (3), et
que la deuxième plaque de support (9) puisse être supportée avec une région d'arête (24) contre la surface de blocage (23) dans sa position soulevée de la première plaque de support (8).

2. Outil manuel selon la revendication 1, **caractérisé en ce que** l'alésage de palier (11) de la deuxième plaque de support (9) du deuxième levier manuel (3) supporté sur le goujon d'articulation (10) est pourvu du côté extérieur d'un élargissement d'alésage (21) élargi radialement, recevant la bride annulaire (12) du goujon d'articulation (10) .

3. Outil manuel selon la revendication 1, **caractérisé en ce que** la surface de blocage (23) est située dans la région d'un plan dans lequel se trouve la surface de contact intérieure (18) de la deuxième plaque de support (9) du deuxième levier manuel (3) tournée vers la première plaque de support (8), lorsque la deuxième plaque de support (9) se trouve sur le goujon d'articulation (10) dans sa position la plus éloignée de la première plaque de support (8) du premier levier manuel (2).

4. Outil manuel selon la revendication 1 ou 3, **caractérisé en ce que** la surface de blocage (23) est pourvue d'une arête guidage oblique (25).

5. Outil manuel selon l'une quelconque des revendications 1 à 4, **caractérisé en ce que** les plaques de support (8, 9) présentent des formes de surfaces au moins approximativement correspondantes et présentent essentiellement la même épaisseur.

6. Outil manuel selon l'une quelconque des revendications 2 à 5, **caractérisé en ce que** dans la région aux alentours de l'élargissement d'alésage (21) est prévue une nervure annulaire périphérique (26), saillant dans la direction du goujon d'articulation (10), dans laquelle se prolonge l'élargissement d'alésage (21), **en ce que** la nervure annulaire (26) se termine dans la région de la bride annulaire (12) du goujon d'articulation (10) lorsque les plaques de support (8, 9) sont situées l'une sur l'autre.
